# EUROPEAN PATENT SPECIFICATION

(11) **EP 1 279 375 B1**
(45) Date of publication and mention of the grant of the patent: **15.03.2006**
(21) Application number: 02255226.9
(22) Date of filing: 26.07.2002
(51) Int. Cl.: A61B 18/22

(54) **Portable laser system**
Tragbares Lasersystem
Système laser portable

(30) Priority: 26.07.2001 GB 0118209
(43) Date of publication of application: 29.01.2003
(73) Proprietor: Carl Zeiss Meditec AG, 07745 Jena (DE)
(72) Inventor: Colles, Michael John, Biggar, Scotland (GB); Silk, Graham David, Kinross, KY13 0NX Scotland (GB); Elder, David Michael, Edinburgh EH5 2EB, Scotland (GB); Thomas, Richard Jolyon, Fife KY11 9LA, Scotland (GB); Lynch, Bruce Robert, Glasgow, G12 0SE, Scotland (GB)
(74) Representative: Murnane, Graham John

(56) References cited:
- EP-A- 0 363 221
- EP-A- 0 473 987
- US-A- 5 553 629
- US-A- 5 575 789
- US-A- 5 748 657

## Description

The present invention relates to portable laser systems for medical applications.

Many types of medical laser systems are known. Such systems enable a laser light beam to be applied locally for treatment of a patient. Examples of the uses of laser light beams include the fields of dentistry, skin treatment and surgical operations. Generally, all of these laser systems are of such size and weight that they cannot be considered to be portable. Considerable effort and time is required to transfer the laser system from one location to another and prepare the system for use. All of the known and universally accepted medical laser systems are powered from an industrial (380 to 415 V) or a standard (220 to 240 V) mains supply.. Therefore these systems cannot be used for remote applications, such as veterinary applications where a mains supply is not available.

US 5553629, upon which the two part form of claim 1 is based, describes a portable medical laser pack system.

It is an object of the present invention to provide a medical laser system that is of such size and weight as to be conveniently portable. It is a further object of the present invention to provide a system that may be powered by a battery.

The invention is defined by appended claim 1. Further preferable embodiments are defined in the dependent claims.

Preferably the control means is adapted to prevent the supply of power to at least the light generating means when the laser system has not been operated for a predetermined period of time.

Preferably the laser system is a diode laser system. Preferably the light generating means includes one diode. The diode may have a wavelength of around 635 nm, or it may have a wavelength of around 690 nm. Alternatively the diode may have a wavelength of between 770 nm and 1000 nm. Alternatively the light generating means includes two diodes. The diodes may have wavelengths of around 635 and around 470 nm. Alternatively a diode or diodes of other wavelengths could be substituted.

Preferably the power output of the or each diode is around 120 mW. Alternatively the power output of the or each diode is around 10 W. Alternatively a diode or diodes of a different power output could be substituted.

Preferably the battery is rechargeable. Preferably the battery is rated at 12 Volts or less. Alternatively the battery is rated at between 12 Volts and 16 Volts. Alternatively the battery is rated at between 16 Volts and 24 Volts. Preferably the control means comprises a driver circuit adapted to allow the light generating means to generate a laser output using a voltage of 12 Volts or less. Preferably the control means is further adapted to control the charging of the battery.

Preferably the control means further comprises a data output means. Preferably the data output means comprises a screen for outputting operational settings and performance characteristics to the user. Preferably the screen is a touch screen adapted to allow the user to input or modify various operational settings. The data output means may further comprise a speaker and a speaker driver circuit. The control means may be adapted to transmit a signal to the driver circuit when one or more operational conditions exceed a predetermined value.

Preferably the light transmission means comprises standard optical fibre telecommunication connectors. Preferably the detachable laser applicator is a disposable hand apparatus.

Preferably, the safety means further comprises a footswitch. Preferably the footswitch is adapted to selectively prevent or allow the supply of power to at least the light generating means.

Preferably the housing is substantially made from moulded plastic. Preferably the housing has the form of a carrying case.

An embodiment of the invention will now be described, by way of example only, with reference to the accompanying figures, where:
Fig. 1 is an exploded perspective view of a portable laser system;
Fig. 2 is a perspective view of a component of the portable laser system of Fig. 1;
Fig. 3 is another perspective view of a component of the portable laser system of Fig. 1;
Fig. 4 is an exploded perspective view of a sub-assembly of the portable laser system of Fig. 1;
Fig. 5 is another exploded perspective view of the sub-assembly of Fig. 4;
Fig. 6 is a block diagram of the portable laser system of Fig. 1; and
Fig. 7 is a circuit diagram of a component of the portable laser system of Fig. 1.

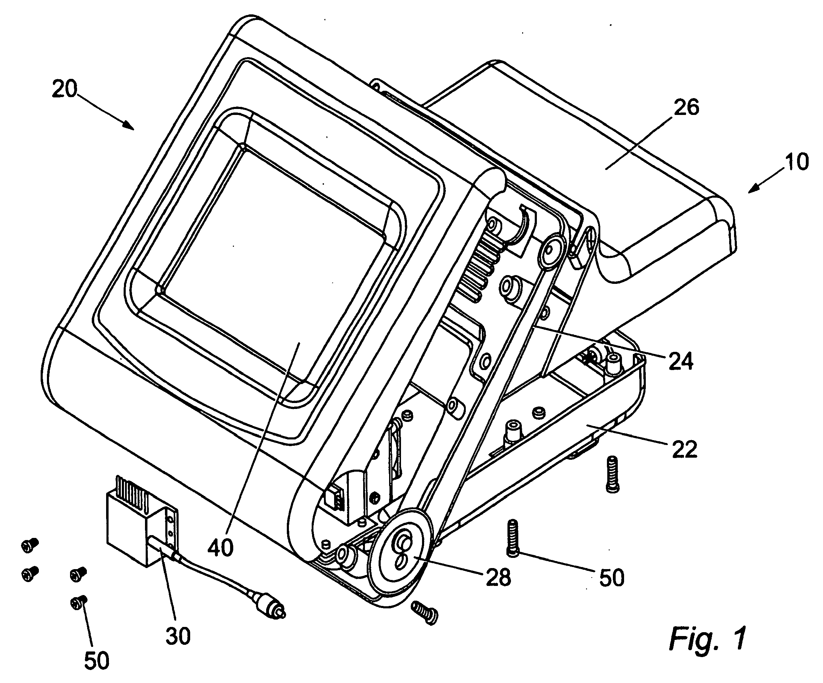

Referring to Fig. 1, there is shown a portable laser system 10 that includes light generating means and control means housed within a housing 20. The light generating means comprises a HPD diode laser 30. Typically, the diode has a wavelength of 635 nm and an output power of 120 mW. Alternatively the diode has a wavelength of between 770 and 1000 nm and an output power of 10 W. The housing 20 comprises a base shell 22, a front top shell 24 and a rear top shell 26, all made from moulded plastic. The control means comprises data output means which comprises a touch screen assembly 40 mounted on the front top shell 24.

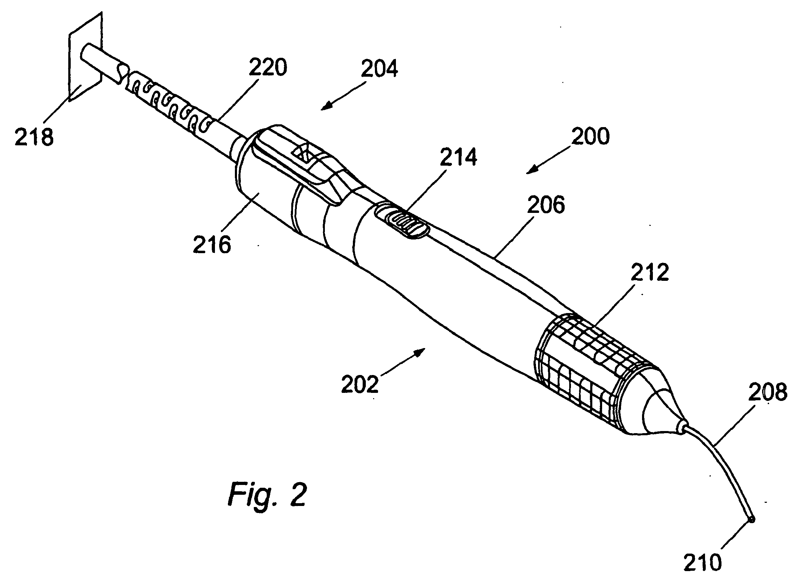

The various portions of the housing 20 are attached to each other in a conventional manner such as press fitting and screws 50. The touch screen assembly 40 is fixed to the front top shell 24 via two mounting discs 28. Located at one of mounting discs 24 is an on/off switch 42. Located at the other mounting disc 28 is a socket 44. This socket is a typical optical fibre telecommunication socket and is adapted to allow the connection of a detachable laser applicator 200. A typical laser applicator 200 is shown in Fig. 2.

The laser applicator 200 comprises a detachable hand-piece 202 connected to an optical coupler 204. The hand-piece 202 includes a housing 206 that contains an optical carrier (not shown). The optical carrier is connected to a flexible elongate sleeve 208 located at one end of the housing 206 and which supports a light emitter 210. The housing 206 includes a gripping portion 212 and a switch 214 for selectively attaching and detaching the hand-piece 202 to the optical coupler 204 at the other end of the housing 206. The optical coupler 204 comprises a socket 216 for receiving the hand-piece 202, a male connector 218 for connection to the light generating means and cabling 220 connecting the socket 216 and male connector 218.

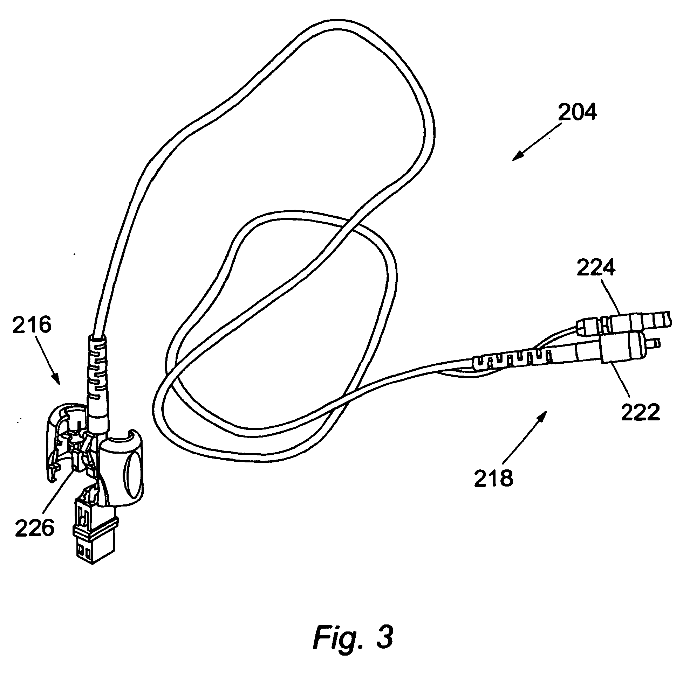

Fig. 3 shows the optical coupler in more detail. Connection of the optical coupler to the light generating means comprises an optical connection 222 and an electrical connection 224.

Safety means are provided such that light may only be transmitted from the light generating means when the optical coupler 204 is connected via the male connector 218. If either the optical connection 222 or the electrical connection 224 are not present, the laser transmission circuit is open and light will not be emitted. Also, light may only be transmitted from the optical coupler 204 if the optical coupler 204 is connected to the hand-piece 202. The socket 216 houses a micro switch 226 which is open when the laser applicator 200 is disconnected from the light generating means, thus preventing the transmission of light.

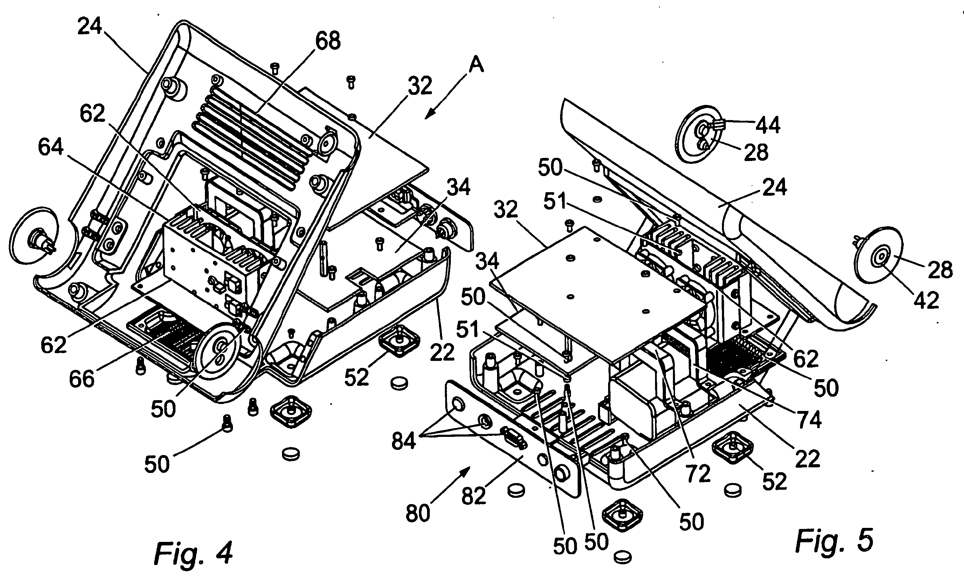

Fig. 4 is the same view as Fig. 1 except that the diode laser 30, touch screen assembly 40 and rear top shell 26 are not shown.

The control means further comprises electronic processing means 32 and a diode laser driver circuit 34. These components are provided as Printed Circuit Boards (PCBs) and are mounted on the base shell 22 using screws 50 and washers 51.

Also mounted on the base shell 22 is a cooling fan unit 60 and a heat sink assembly 62. When the laser system 10 is in use, the impeller fans of the cooling fan unit 60 draw air that has been heated by the PCBs 32, 34 and direct this air towards the heat sink assembly 62. The heat sink assembly 62 is made of a suitable material and has a number of fins 64 of large surface area such that the heat is conducted and then dissipated. Air is then directed downwards by the heat sink assembly 62 so that it exits the housing 20 via an outlet vent 66. The vacuum created by drawing the heated air away from the PCBs 32, 34 causes air at ambient temperature to enter the housing 20 via an inlet vent 68.

On the back face of the heat sink assembly 62 are provided tapped holes 63 for mounting the diode laser 30 (not shown in Fig. 4) using screws 50. The holes 63 are arranged such that the diode laser 30 may be mounted in a horizontal or vertical position.

Four feet 52 are fixed to the underside of the base shell 22. Each foot 52 includes an adhesive portion 54 for securely positioning the laser system 10 on a supporting surface (not shown).

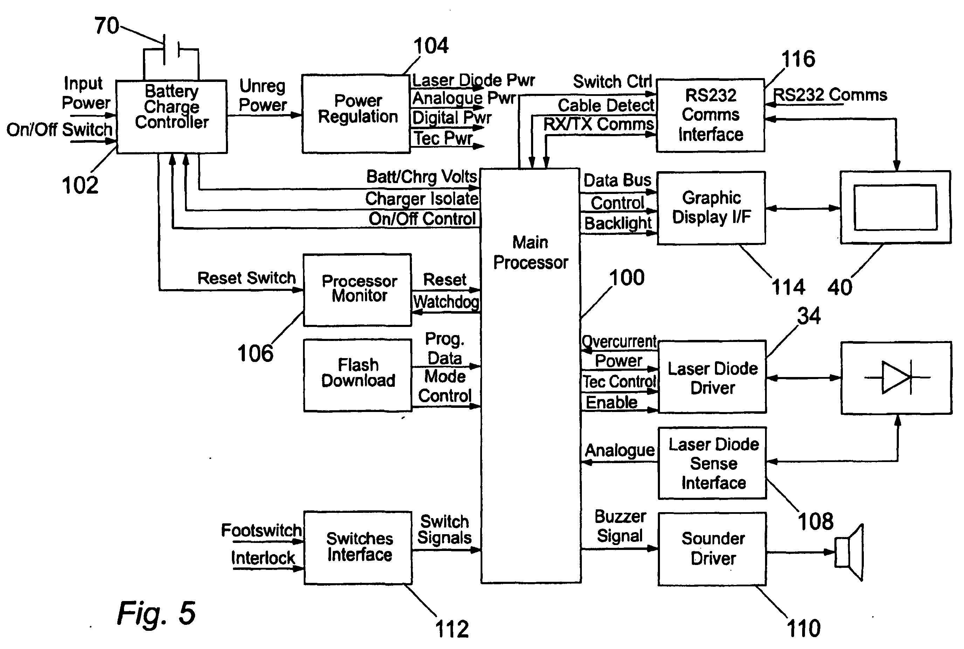

Fig. 5 shows the laser system 10 as viewed from arrow 'A' in Fig. 4.

The power supply means is also mounted on the base shell 22. The power supply means comprises a battery assembly. This battery assembly comprises a rechargeable battery 70 which is fixed to the base shell using battery straps 72. Typically, the battery is a Lithium-Iron cell rated at 5.5 Ah. The battery has a voltage rating of 12 V. Alternatively a battery having a voltage rating of 16 V may be used, such as when diodes of a greater output power are used. To protect the battery from shock or vibrational damage, PVC foam 74 is provided between the battery 70 and the battery straps 72.

At the rear of the laser system 10 is located a back panel assembly 80. The panel assembly consists of a plate 82 with a number of apertures. Mounted within these apertures are a number of sockets 84. These sockets 84 are connected to the electronic processing means 32 via wiring (not shown). The sockets 84 allow connection to external devices (not shown) which may include an on/off footswitch or a Personal Computer (PC).

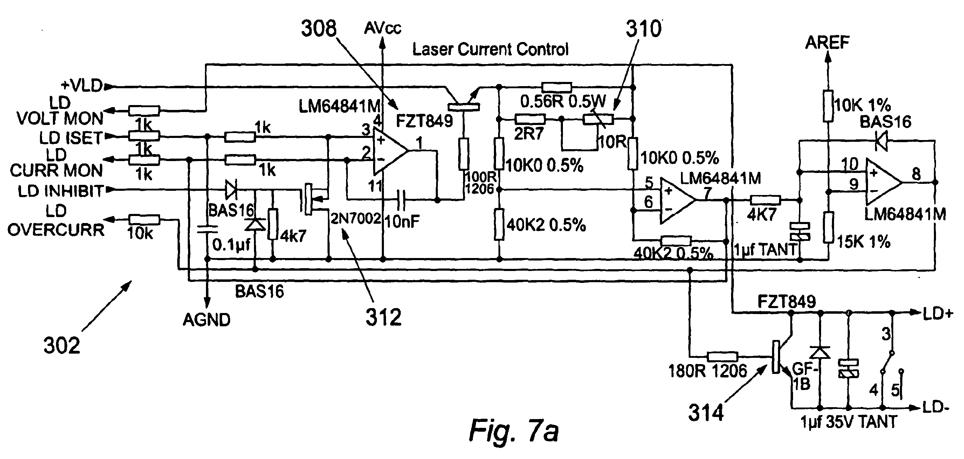

Fig. 6 is a block diagram of the electronic circuit provided by the PCBs 32, 34. The processing is performed by a main processor 100. Power may be supplied to the processor 100 from the battery 70, or from mains power, via charge controller circuitry 102. The controller 102 is adapted to select mains power as the power source when a mains source is present and, at the same time, to direct a proportion of this power to the rechargeable battery 70.

Power is also supplied from the charge controller 102 to the diode laser 30, the diode driver circuit 34 and to the cooling fan unit 60 via a power regulation unit 104.

Power is also supplied to a processor monitor 106. The processor monitor 106 includes a timer circuit and is adapted to prevent the supply of power to the diode driver circuit (and hence the diode laser 30) if the laser system has not been operated for a predetermined period of time. The processor monitor 106 also prevents the supply of power to the processor 100. Therefore, no component of the laser system 10 is drawing power from the battery 70 and so power is conserved. The laser system is then termed as being in a "sleep mode". The user may reset the laser system 10 to an operating mode by pressing the on/off switch 42.

The processor 100 provides control signals to the diode driver circuit 34. Signals are also received by the processor 100 from the driver circuit 34 via a sense interface 108. These signals are generated by sensors that measure operational conditions such as temperature (at the heat sink assembly 62 and at the diode laser), light intensity and voltage and current levels in the driver circuit 34. The processor 100 is adapted to compare the values of these measured conditions with predefined values and take action when measured values exceed the predefined values. The action will depend on the value that is excessive and can vary from providing more power to the cooling fan unit 60 to preventing power being supplied to the driver circuit 34. In the latter case, and also for less serious cases, a signal may be sent to a sounder driver 110 to generate an audible noise to warn the user.

The user may also prevent power being supplied to the driver circuit 34. This can be achieved by disconnecting the laser applicator 200 from the light generating means, as described above. Additionally, a footswitch 112 is provided which, when released, sends a signal to the processor 100 to disconnect the driver circuit 34.

The processor 100 provides data and control signals to the touch screen 40 via a graphic display interface 114. The graphic display interface 114 formats data from the processor 100 so that it may be displayed on the touch screen 40, and also receives data inputted by the user on the touch screen 40 and formats the data so that it may be received by the processor 100. The data consists of the operational parameters and conditions of the laser system 10.

The touch screen is connected to the processor 100 by a standard RS232 connection port 116. Similar ports are available at the back panel assembly 80 for connecting the processor 100 to external devices such as PCs (not shown).

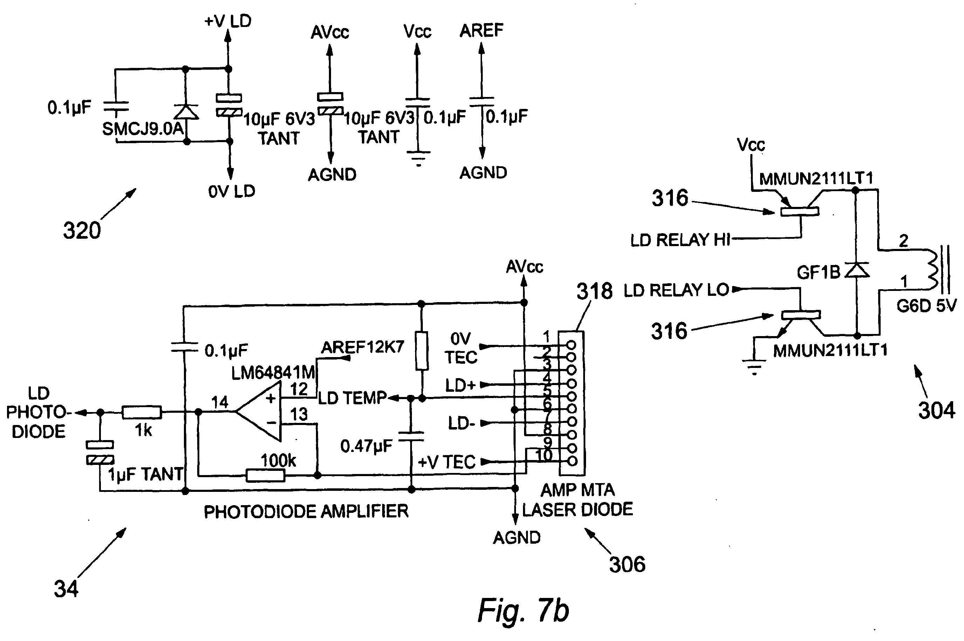

Fig. 7 shows the electronic circuit of the driver circuit 34. It should be appreciated that the design of the circuit allows the use of a low supply voltage, not achieved by the circuitry of conventional systems. Furthermore, the circuit represents a far more simplified design, dedicated to the medical application for which it is intended. This leads to a reduction in the space required and the weight of the apparatus, which is highly desirable in a portable system. The circuit may be considered as three distinct sections 302, 304, 306.

The current driver circuit 302 controls the current that is supplied to the diode laser 30. As the power available to the diode laser 30 is more proportional to the current than the supply voltage, current control allows for more effective control of the output power of the diode laser 30.

Within the current driver circuit 302, a transistor 308 controls the current through the diode laser 30. A variable resistor 310 allows calibration of this current. Also present is a transistor 312 that inhibits the input signal reaching the diode laser 30 when an inhibit signal is present. This signal is removed once the fire up sequence has been completed and the diode laser 30 has reached a suitable operating temperature. Another transistor 314 represents a 'crowbar' clamping transistor that limits the drive current to protect the diode laser 30, typically to 750 mA.

Diode protection is also performed by a second section 304 of the driver circuit 34. Two transistors 316 form a shorting relay driver operating in a 'fail safe' mode.

The third section 306 of the circuit is more directly responsible for driving the diode laser 30. A connecting component 318 represents the input connections to the diode laser 30. The input signals include LD+ and LD- which are the output signals from the current driver circuit 302.

A number of capacitors 320 are also present within the circuit to smooth the circuit current and voltage. These capacitors are of standard and polarised construction.

Modifications and variations to the invention described above are possible without departing from the scope of the invention as defined by the following claims.

## Claims

1. A portable laser system (10) for medical applications comprising:
light generating means (30);
control means;
power supply means adapted to supply power to the light generating means (30) and the control means, wherein the power supply means comprises a battery (70);
and
laser application means (200) comprising a detachable laser applicator (202) and light transmission means,
**characterised in that**
the laser application means (200) is optically and electrically connectable to the light generating means (30); and **in that** the system further comprises
safety means adapted to prevent light being transmitted along the light transmission means when the laser applicator (202) is either optically or electrically detached.

2. A portable laser system (10) according to Claim 1, wherein the safety means comprises a switch (226) which is open when the laser applicator (202) is either optically or electrically detached such that the transmission of light is prevented.

3. A portable laser system (10) according to Claim 1 or 2, wherein the control means is adapted to prevent the supply of power to at least the light generating means (30) when the laser system (10) has not been operated for a predetermined period of time.

4. A portable laser system (10) according to Claim 3, including a processor (100), and wherein the control means is further adapted to prevent the supply of power to the processor (100) when the laser system (10) has not been operated for a predetermined period of time.

5. A portable laser system (10) according to any preceding claim, wherein the laser system is a diode laser system, and wherein the light generating means (30) includes at least one diode.

6. A portable laser system (10) according to Claim 5, wherein the control means includes a transistor (308) for controlling the current through the diode.

7. A portable laser system (10) according to Claim 5 or 6, wherein the diode has a wavelength of between 635 nm and 690 nm.

8. A portable laser system (10), according to Claim 5 or 6, wherein the diode has a wavelength of between 770 nm and 1000 nm.

9. A portable laser system (10) according to any of Claims 5 to 8, wherein the light generating means (30) includes two diodes.

10. A portable laser system (10) according to any of Claims 5 to 9, wherein the power output of the or each diode is around 120 mW.

11. A portable laser system (10) according to any of Claims 5 to 9, wherein the power output of the or each diode is around 10 W.

12. A portable laser system (10) according to any previous claim, wherein the battery (70) has a rating of 12 Volts or less.

13. A portable laser system (10) according to any of Claims 1 to 11, wherein the battery (70) has a rating of between 12 Volts and 16 Volts.

14. A portable laser system (10) according to any previous claim, wherein the control means comprises data output means.

15. A portable laser system (10) according to Claim 14, wherein the data output means comprises a screen (40) for outputting operational settings and/or performance characteristics to the user.

16. A portable laser system (10) according to Claim 15, wherein the screen is a touch screen (40) adapted to allow the user to input or modify the operational settings.

17. A portable laser system (10) according to Claim 4, wherein the light transmission means comprises standard optical fibre telecommunication connectors.

18. A portable laser system (10) according to Claim 4 or Claim 17, wherein the detachable laser applicator (202) is a disposable hand apparatus.

19. A portable laser system (10) according to any of Claims 4, 17 or 18, wherein the safety means comprises a footswitch (112) adapted to selectively prevent or allow the supply of power to at least the light generating means (30).

## Revendications

1. Un système laser portable (10) pour des applications médicales comportant :
un moyen de génération de lumière (30) ;
un moyen de contrôle ;
un moyen d'alimentation électrique adapté pour alimenter en électricité le moyen de génération de lumière (30) et le moyen de contrôle, dans lequel le moyen d'alimentation électrique comporte une batterie (70) ;
et
un moyen d'application laser (200) comportant un applicateur laser détachable (202) et un moyen de transmission de lumière,
**caractérisé en ce que**
le moyen d'application laser (200) peut être connecté de façon optique et électrique au moyen de génération de lumière (30) ; et **en ce que** le système comporte de plus
un moyen de sécurité adapté pour empêcher que de la lumière soit transmise le long du moyen de transmission de lumière lorsque l'applicateur laser (202) est détaché soit de façon optique, soit de façon électrique.

2. Un système laser portable (10) selon la revendication 1, dans lequel le moyen de sécurité comporte un commutateur (226) qui est ouvert lorsque l'applicateur laser (202) est détaché soit de façon optique, soit de façon électrique de sorte que la transmission de lumière soit empêchée.

3. Un système laser portable (10) selon la revendication 1 ou la revendication 2, dans lequel le moyen de contrôle est adapté pour empêcher qu'au moins le moyen de génération de lumière (30) soit alimenté en électricité lorsque le système laser (10) n'a pas fonctionné pendant une période de temps prédéterminée.

4. Un système laser portable (10) selon la revendication 3, comprenant un processeur (100), et dans lequel le moyen de contrôle est adapté plus avant pour empêcher que le processeur (100) soit alimenté en électricité lorsque le système laser (10) n'a pas fonctionné pendant une période de temps prédéterminée.

5. Un système laser portable (10) selon n'importe quelle revendication précédente, dans lequel le système laser est un système laser à diode, et dans lequel le moyen de génération de lumière (30) comprend au moins une diode.

6. Un système laser portable (10) selon la revendication 5, dans lequel le moyen de contrôle comprend un transistor (308) destiné à contrôler le courant à travers la diode.

7. Un système laser portable (10) selon la revendication 5 ou la revendication 6, dans lequel la diode a une longueur d'onde d'entre 635 nm et 690 nm.

8. Un système laser portable (10) selon la revendication 5 ou la revendication 6, dans lequel la diode a une longueur d'onde d'entre 770 nm et 1 000 nm.

9. Un système laser portable (10) selon n'importe lesquelles des revendications 5 à 8, dans lequel le moyen de génération de lumière (30) comprend deux diodes.

10. Un système laser portable (10) selon n'importe lesquelles des revendications 5 à 9, dans lequel la puissance de sortie de la diode ou de chaque diode est d'environ 120 mW.

11. Un système laser portable (10) selon n'importe lesquelles des revendications 5 à 9, dans lequel la puissance de sortie de la diode ou de chaque diode est d'environ 10 W.

12. Un système laser portable (10) selon n'importe quelle revendication précédente, dans lequel la batterie (70) a un calibre de 12 volts ou moins.

13. Un système laser portable (10) selon n'importe lesquelles des revendications 1 à 11, dans lequel la batterie (70) a un calibre d'entre 12 volts et 16 volts.

14. Un système laser portable (10) selon n'importe quelle revendication précédente, dans lequel le moyen de contrôle comporte un moyen de sortie de données.

15. Un système laser portable (10) selon la revendication 14, dans lequel le moyen de sortie de données comporte un écran (40) destiné à sortir des paramètres de fonctionnement et/ou des caractéristiques de performance à l'intention de l'utilisateur.

16. Un système laser portable (10) selon la revendication 15, dans lequel l'écran est un écran tactile (40) adapté pour permettre à l'utilisateur d'entrer ou de modifier les paramètres de fonctionnement.

17. Un système laser portable (10) selon la revendication 4, dans lequel le moyen de transmission de lumière comporte des connecteurs de télécommunication de fibre optique standard.

18. Un système laser portable (10) selon la revendication 4 ou la revendication 17, dans lequel l'applicateur laser détachable (202) est un appareil à main jetable.

19. Un système laser portable (10) selon n'importe lesquelles des revendications 4, 17 ou 18, dans lequel le moyen de sécurité comporte un interrupteur au pied (112) adapté pour empêcher ou permettre de façon sélective qu'au moins le moyen de génération de lumière (30) soit alimenté en électricité.

## Patentansprüche

1. Ein tragbares Lasersystem (10) für medizinische Anwendungen, das Folgendes beinhaltet:
ein Lichterzeugungsmittel (30);
ein Steuermittel;
ein Energieversorgungsmittel, das angepasst ist, um das Lichterzeugungsmittel (30) und das Steuermittel mit Energie zu versorgen, wobei das Energieversorgungsmittel eine Batterie (70) beinhaltet;
und
ein Laserappliziermittel (200), das einen abtrennbaren Laserapplikator (202) und ein Lichtübertragungsmittel beinhaltet,
**dadurch gekennzeichnet, dass**
das Laserappliziermittel (200) optisch und elektrisch mit dem Lichterzeugungsmittel (30) verbunden werden kann; und **dadurch**, dass das System ferner Folgendes beinhaltet
ein Sicherheitsmittel, das angepasst ist, um die Lichtübertragung entlang dem Lichtübertragungsmittel zu verhindern, wenn der Laserapplikator (202) entweder optisch oder elektrisch abgetrennt ist.

2. Tragbares Lasersystem (10) gemäß Anspruch 1, wobei das Sicherheitsmittel einen Schalter (226) beinhaltet, der geöffnet ist, wenn der Laserapplikator (202) entweder optisch oder elektrisch abgetrennt ist, so dass die Lichtübertragung verhindert wird.

3. Tragbares Lasersystem (10) gemäß Anspruch 1 oder 2, wobei das Steuermittel angepasst ist, um das Versorgen von mindestens dem Lichterzeugungsmittel (30) mit Energie zu verhindern, wenn das Lasersystem (10) während eines bestimmten Zeitraums nicht bedient worden ist.

4. Tragbares Lasersystem (10) gemäß Anspruch 3, das einen Prozessor (100) umfasst, und wobei das Steuermittel ferner angepasst ist, um das Versorgen des Prozessors (100) mit Energie zu verhindern, wenn das Lasersystem (10) während eines bestimmten Zeitraums nicht bedient worden ist.

5. Tragbares Lasersystem (10) gemäß einem der vorhergehenden Ansprüche, wobei das Lasersystem ein Diodenlasersystem ist, und wobei das Lichterzeugungsmittel (30) mindestens eine Diode umfasst.

6. Tragbares Lasersystem (10) gemäß Anspruch 5, wobei das Steuermittel einen Transistor (308) zum Steuern des Stroms durch die Diode umfasst.

7. Tragbares Lasersystem (10) gemäß Anspruch 5 oder 6, wobei die Diode eine Wellenlänge von zwischen 635 nm und 690 nm aufweist.

8. Tragbares Lasersystem (10) gemäß Anspruch 5 oder 6, wobei die Diode eine Wellenlänge von zwischen 770 nm und 1 000 nm aufweist.

9. Tragbares Lasersystem (10) gemäß einem der Ansprüche 5 bis 8, wobei das Lichterzeugungsmittel (30) zwei Dioden umfasst.

10. Tragbares Lasersystem (10) gemäß einem der Ansprüche 5 bis 9, wobei die Leistungsabgabe der oder jeder Diode ungefähr 120 mW beträgt.

11. Tragbares Lasersystem (10) gemäß einem der Ansprüche 5 bis 9, wobei die Leistungsabgabe der oder jeder Diode ungefähr 10 W beträgt.

12. Tragbares Lasersystem (10) gemäß einem der vorhergehenden Ansprüche, wobei die Batterie (70) eine Nennleistung von 12 Volt oder weniger aufweist.

13. Tragbares Lasersystem (10) gemäß einem der Ansprüche 1 bis 11, wobei die Batterie (70) eine Nennleistung von zwischen 12 Volt und 16 Volt aufweist.

14. Tragbares Lasersystem (10) gemäß einem der vorhergehenden Ansprüche, wobei das Steuermittel ein Datenausgabemittel beinhaltet.

15. Tragbares Lasersystem (10) gemäß Anspruch 14, wobei das Datenausgabemittel einen Bildschirm (40) zum Ausgeben von Bedienungseinstellungen und/oder Kennwerten des Leistungsverhaltens an den Anwender beinhaltet.

16. Tragbares Lasersystem (10) gemäß Anspruch 15, wobei der Bildschirm ein Berührungsblidschirm (40) ist, der angepasst ist, um dem Anwender das Eingeben oder Modifizieren von Bedienungseinstellungen zu ermöglichen.

17. Tragbares Lasersystem (10) gemäß Anspruch 4, wobei das Lichtübertragungsmittel handelsübliche Lichtwellenleiter-Telekommunikationsstecker beinhaltet.

18. Tragbares Lasersystem (10) gemäß Anspruch 4 oder Anspruch 17, **wobei der abtrennbare Laserapplikator (202) ein Einweghandgerät** ist.

19. Tragbares Lasersystem (10) gemäß einem der Ansprüche 4, 17 oder 18, wobei das Sicherheitsmittel einen Fußschalter (112) beinhaltet, der angepasst ist, um das Versorgen von mindestens dem Lichterzeugungsmittel (30) mit Strom wahlweise zu verhindern oder zu erlauben.
